(19) Europäisches Patentamt
European Patent Office
Office européen des brevets

(11) Publication number: **0 122 419**
**A2**

(12) # EUROPEAN PATENT APPLICATION

(21) Application number: 84102273.4

(22) Date of filing: 02.03.84

(51) Int. Cl.³: C 07 D 403/10
C 07 D 409/14
//C07D233/00

(30) Priority: 21.03.83 DE 3310197

(43) Date of publication of application: 24.10.84 Bulletin 84/43

(84) Designated Contracting States: AT BE CH DE FR GB IT LI LU NL SE

(71) Applicant: A. Nattermann & Cie. GmbH
Nattermannallee 1
D-5000 Köln 30(DE)

(72) Inventor: Hilboll, Gerd, Dr.
Dehmelstrasse 36
D-5000 Köln 30(DE)

(72) Inventor: Borbe, Harald O., Dr.
Frechener Weg 30
D-5000 Köln 40(DE)

(72) Inventor: Doppelfeld, Ille-Stephanie
Im Bruchfeldchen 35
D-5010 Bergheim-Glessen(DE)

(72) Inventor: Prop, Gerrit, Dr.
Anemonenweg 23
D-5024 Pulheim(DE)

(74) Representative: Redies, Bernd, Dr. rer. nat. et al,
Redies, Redies, Türk & Gille Brucknerstrasse 20
D-4000 Düsseldorf 13(DE)

(54) **Substituted 3-mercaptopyridazines, their 3-alkylthio derivatives and processes for their preparation.**

(57) The invention relates to new substituted 3-mercaptopyridazines and their 3-alkylthio derivatives of the formula 1

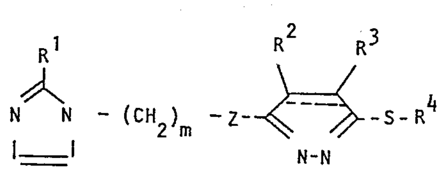

and processes for their preparation.

EP 0 122 419 A2

The present invention relates to substituted 3-mercapto-pyridazines and their 3-alkylthio derivatives of the general formula I

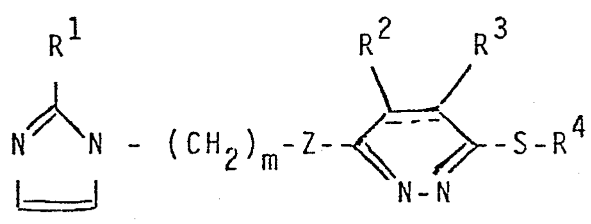

I

in which $R^1$, $R^2$, $R^3$, $R^4$ can be the same or different and independently signify hydrogen or a $C_{1-4}$-lower alkyl residue, m stands for 0 or a whole number from 1 to 5, Z signifies 1.4-phenylene or 2.5- or 2.4-thienylene, and $\overline{----}$ signifies a double or a single bond between two carbon atoms; pharmaceutically acceptable salts of compounds of the formula I with inorganic or organic acids, e.g. hydrochlorides, acetates, fumarates, citrates and benzoates are also included.

Preferred are those compounds of formula I wherein $R^4$ is hydrogen and m is cero or 1 and $\overline{----}$ is a single or double bond.

The compounds of the formula I with $R^4$ = hydrogen, in which ---- signifies a single bond, are predominantly in their tautomeric form as 4,5-Dihydro-3-(2H)-pyridazinethiones.

The compounds of the formula I with $R^4$ = hydrogen, in which ---- signifies a double bond, exists in equilibrium with the corresponding tautomeric 3(2H)-pyridazinethiones.

The 4.5-dihydro-3-mercapto-pyridazines of the formula I ($R^4$ = hydrogen, ---- signifies a single bond) possess a centre of chirality at positions 4 or 5 of the pyridazine ring, when the substituents $R^2$ and/or $R^3$ are other than hydrogen, and can thus be present either as a racemic mixture or in the form of the enantiomers. If separation of the racemic mixture is desired, it is usefully carried out by methods known per se with an optically active acid, e.g. dibenzoyltartaric acid or camphor-10-sulphonic acid via the formation of diastereomeric salts or by chromatography on optically active column material.

The compounds of the formula I have valuable pharmacological properties.

Compounds of the invention are for example:
4.5-Dihydro-6-[4-(1-imidazolylmethyl)-phenyl]-3-mercapto-pyridazine and its tautomeric thione form
4.5-dihydro-6-{4-[2-(1-imidazolyl)-ethyl]-phenyl}-3-mercapto-pyridazine and its tautomeric thione form
4.5-dihydro-6-{4-[3-(1-imidazolyl)-propyl]-phenyl}-3-mercaptopyridazine and its tautomeric thione form
4.5-dihydro-6-{4-[4-(1-imidazolyl)-butyl]-phenyl}-3-mercaptopyridazine and its tautomeric thione form
4.5-dihydro-6-{4-[5-(1-imidazolyl)-pentyl]-phenyl}-3-mercaptopyridazine and its tautomeric thione form
4.5-dihydro-6-[4-(1-imidazolyl)-phenyl]-3-mercaptopyridazine and its tautomeric thione form

4.5-dihydro-3-mercapto-6-[4-(2-methyl-1-imidazolyl)-phenyl]-pyridazine and its tautomeric thione form
4.5-dihydro-6-[4-(2-ethyl-1-imidazolyl)-phenyl]-3-mercapto-pyridazine and its tautomeric thione form
4.5-dihydro--3-mercapto-6-[4-(2-propyl-1-imidazolyl)-phenyl]-pyridazine and its tautomeric thione form
6-[4-(2-butyl-1-imidazolyl)-phenyl]-4.5-dihydro-3-mercapto-pyridazine and its tautomeric thione form
4.5-dihydro-6-[4-(1-imidazolyl)-phenyl]-3-mercapto-4-methyl-pyridazine and its tautomeric thione form
4.5-dihydro-6-[4-(1-imidazolyl)-phenyl]-3-mercapto-5-methyl-pyridazine and its tautomeric thione form
4.5-dihydro-4-ethyl-6-[4-(1-imidazolyl)-phenyl]-3-mercapto-pyridazine and its tautomeric thione form
4.5-dihydro-5-ethyl-6-[4-(1-imidazolyl)-phenyl]-3-mercapto-pyridazine and its tautomeric thione form
4.5-dihydro-3-mercapto-5-methyl-6-[4-(2-methyl-1-imidazolyl)-phenyl]-pyridazine and its tautomeric thione form
4.5-dihydro-6-[5-(1-imidazolylmethyl)-thien-2-yl]-3-mercaptopyridazine and its tautomeric thione form
4.5-dihydro-6-[5-(1-imidazolyl)-thien-2-yl]-3-mercapto-pyridazine and its tautomeric thione form
4.5-dihydro-6-{5-[2-(1-imidazolyl)-ethyl]-thien-2-yl}-3-mercaptopyridazine and its tautomeric thione form
4.5-dihydro-6-[5-(1-imidazolyl)-thien-2-yl]-3-mercapto-5-methylpyridazine and its tautomeric thione form
4.5-dihydro-6-[4-(1-imidazolylmethyl)-thien-2-yl]-3-mercaptopyridazine and its tautomeric thione form
4.5-dihydro-6-[4-(1-imidazolyl)-thien-2-yl]-3-mercapto-pyridazine and its tautomeric thione form
4.5-dihydro-6-[4-(1-imidazolyl)-thien-2-yl]-3-mercapto-5-methylpyridazine and its tautomeric thione form
6-[4-(1-imidazolylmethyl)-phenyl]-3-mercaptopyridazine and its tautomeric thione form
6-[4-(1-imidazolyl)-phenyl]-3-mercaptopyridazine and its tautomeric thione form

3-mercapto-6-[4-(2-methyl-1-imidazolyl)-phenyl]-pyridazine and its tautomeric thione form
6-[4-(1-imidazolyl)-phenyl]-3-mercapto-4-methylpyridazine and its tautomeric thione form
6-[4-(1-imidazolyl)-phenyl]-3-mercapto-5-methylpyridazine and its tautomeric thione form
3-mercapto-5-methyl-6-[4-(2-methyl-1-imidazolyl)-phenyl]-pyridazine and its tautomeric thione form
6-[5-(1-imidazolylmethyl)-thien-2-yl]-3-mercapto-pyridazine and its tautomeric thione form
6-[5-(1-imidazolyl)-thien-2-yl]-3-mercaptopyridazine and its tautomeric thione form
6-[5-(1-imidazolyl)-thien-2-yl]-3-mercapto-5-methyl-pyridazine and its tautomeric thione form
6-[4-(1-imidazolyl)-thien-2-yl]-3-mercaptopyridazine and its tautomeric thione form
6-[4-(1-imidazolyl)-thien-2-yl]-3-mercapto-5-methyl-pyridazine and its tautomeric thione form
6-[4-(1-imidazolylmethyl)-phenyl]-3-methylthiopyridazine
6-[4-(1-imidazolyl)-phenyl]-3-methylthiopyridazine
6-[4-(1-imidazolyl)-phenyl]-5-methyl-3-methylthio-pyridazine
5-methyl-6-[4-(2-methyl-1-imidazolyl)-phenyl]-3-methylthio-pyridazine
6-[5-(1-imidazolylmethyl)-thien-2-yl]-3-methylthio-pyridazine
6-[5-(1-imidazolyl)-thien-2-yl]-5-methyl-3-methylthio-pyridazine
6-[4-(1-imidazolyl)-thien-2-yl]-5-methyl-3-methylthio-pyridazine
3-ethylthio-6-[4-(1-imidazolyl)-phenyl]-5-methylpyridazine
6-[4-(1-imidazolyl)-phenyl]-5-methyl-3-propylthio-pyridazine
6-[4-(1-imidazolyl)-phenyl]-3-isopropylthio-5-methyl-pyridazine
3-butylthio-6-[4-(1-imidazolyl)-phenyl]-5-methyl-pyridazine

3-butylthio-6-[5-(1-imidazolyl)-thien-2-yl]-5-methylpyridazine

The preparation of the compounds of the invention of formula I takes place by processes known <u>per se</u>. Thus the 4.5-dihydro-3-mercaptopyridazines of the fomrula I ($R^4$ = hydrogen, ---- signifies a single bond) are prepared from 3-oxo-2.3.4.5-tetrahydropyridazines of the formula II

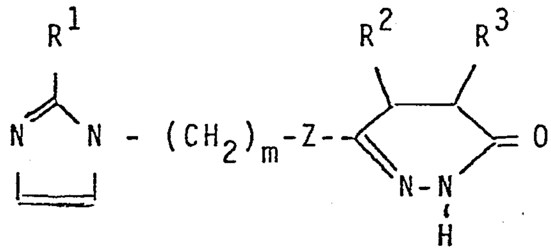

II

in which m, Z $R^1$, $R^2$, $R^3$ have the meanings given in formula I, by reaction with 2.4-bis-(4-methoxyphenyl)-2.4-dithioxo-1.3.2.4-dithiadiphosphetan (Lawesson-Reagent) in a protective gas atmosphere in a suitable organic solvent, e.g. toluene, chloroform, hexamethylphosphoric acid triamide, at temperatures from 60-110°C, in a manner analogous to that described in K Clausen, S.O. Lawesson, Nouv. J. Chim. 4 (1980), 43.
These compounds can also be prepared similarly to the description in E. Klingsberg, D. Papa, J.Am.Chem.Soc. 73 (1951), 4988, by conversion of the compounds of the formula II in a protective gas atmosphere with diphosphorus pentasulphide in a suitable organic solvent, e.g. pyridine, benzene, toluene, at temperatures of 60-110°C.

The preparation of the 3-oxo-2.3.4.5-tetrahydropyridazines of formula II used as starting compounds takes place by reaction of a 4-oxobutyric acid of formula III

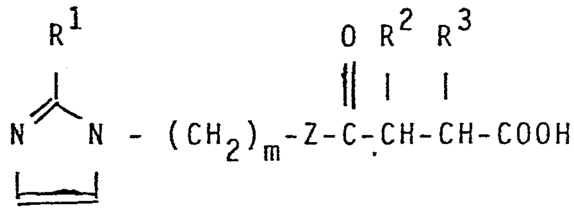

III

in which m, Z, $R^1$, $R^2$, $R^3$ have the meanings given in formula I, or their lower alkyl esters, with hydrazine or its hydrate or a hydrazine salt such as the hydrochloride, hydrogen sulphate, sulphate etc., in aqueous, aqueous/alcoholic or alcoholic media or in organic solvents which are inert under the selcected conditions e.g. dioxan, toluene, dimethylformamide, at temperatures from 60-150°C, preferably at 80-100°C in ethanol or water. Some of these compounds (where m ≠ 0) have been described in EP 71 059 and EP 71 060.

The preparation of the starting compounds of the formula III where m = 0 takes place by methods known per se; Imidazolyl aldehydes of the formula IV (L.M.Sitkina, A.M. Simonov, C.A. 65 (1966) 13686e)

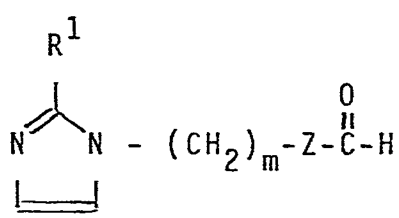

IV (m = 0)

are reacted under the catalytic influence of sodium cyanide (H. Stetter, Angew. Chem. 88 (1976), 695-736) with 2-alkenoic acid nitriles of the formula V

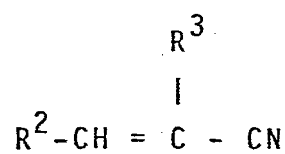

V

to the 4-oxobutyric acid nitriles of the formula VI

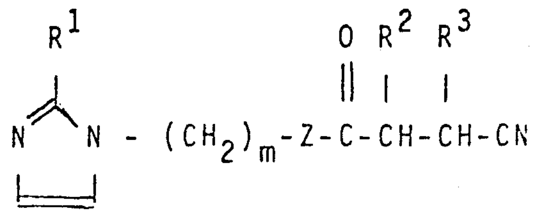

VI (m = 0)

the latter being converted by hydrochloric acid into the 4-oxobutyric acids of the formula III.

If the above described sulphidation is carried out in the presence of an oxidizing agent, e.g. atmospheric oxygen, then the 3-mercaptopyridazines of the formula I ($R^4$ = hydrogen, ---- signifies a double bond) are obtained directly from compounds of the formula II.

These 3-mercaptopyridazines can also be prepared from 2,3-dihydro-3-oxo-pyridazines of the formula VII

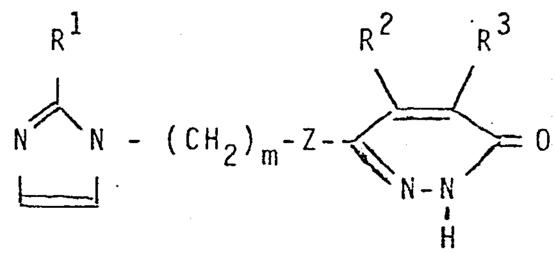

VII

in which m, Z, $R^1$, $R^2$, $R^3$ have the meanings given in formula I, by both of the described sulphidation processes by reaction with Lawesson reagent or diphosphorus pentasulphide.

The preparation of the compounds of the formula VII takes place by methods known *per se* (v. J. Heterocycl. Chem. 15 (1978), 881) by oxidation of the compounds of the formula II with bromine or sodium m-nitrobenzene sulphonate.

The 3-alkylthio-pyridazines of the formula I ($R^4$ = alkyl, ---- signifies a double bond) are prepared by reaction of the 3-mercaptopyridazines of the formula I ($R^4$ = hydrogen, ---- signifies a double bond) with an alkylating agent of the formual VIII

$$X - R^4 \qquad \text{VIII}$$

in which $R^4$ signifies a $C_{1-4}$-lower alkyl residue and X stands form a leaving group, e.g. chloride, bromide, iodide, methylsulphate, tosylate, in a suitable organic solvent e.g. dimethylformamide, dimethylsulphoxide, hexamethylphosphoric acid triamide, with optional use of an auxiliary base, e.g. sodium hydride, potassium carbonate, at temperatures from 60-150°C.

These 3-alkylthiopyridazines of the formual I can be prepared likewise by alkylation of the 4.5-dihydro-3-mercaptopyridazines of the formula I ($R^4$ = hydrogen, ---- signifies a single bond) in the presence of an oxidizing agent, e.g. atmospheric oxygen, using the alkylating agents of the formula VIII.

Examples of the alkylating agents of the formula VIII are as follows:
Methyl iodide, ethyl bromide, 1-bromopropane, 1-chloropropane, 1-chlorobutane, 1-bromobutane, 2-bromopropane, 2-bromobutane, 2-methyl-1-bromopropane, dimethyl sulphate, methyl tosylate.

The processes described are illustrated by the following formula diagram:

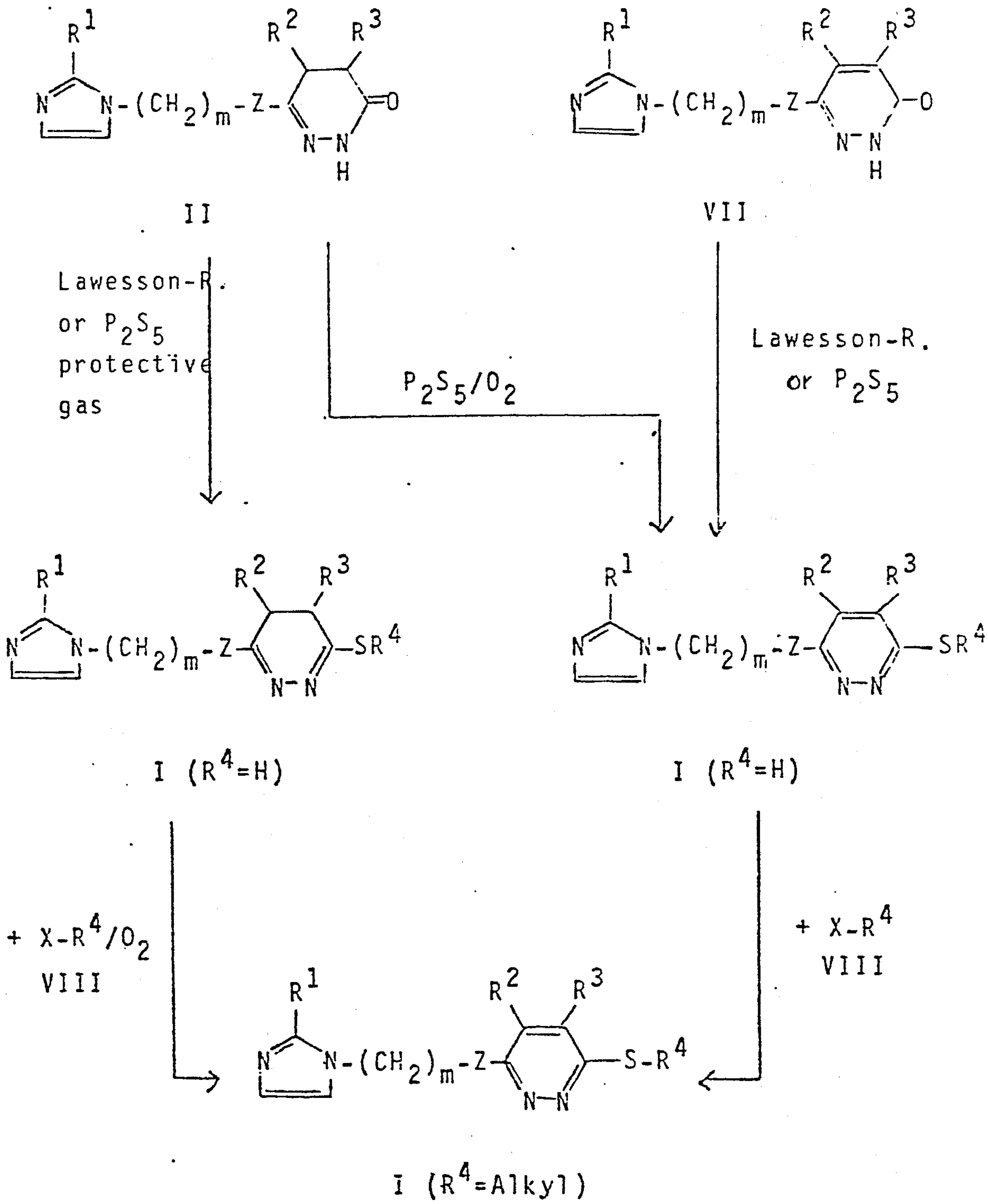

The acid addition salts of compounds of the formula I with inorganic or organic acids can be prepared by mixing the imidazolyl compound of origin with the corresponding acids in aqueous, aqueous/organic (e.g. alcohol/water) or organic media, e.g. alcohols, alcohol/ether mixtures or ether/petroleum ether mixtures at temperatures between 0 and 100°C.

The compounds of formula I according to the invention are distinguished by thrombocyte aggregation inhibiting and especially by blood pressure lowering properties. They are suitable for use in particular as anti-hypertensives and furthermore for the prophylaxis and therapy of thromboembolic illnesses.

The present invention also relates to a process for the treatment of high blood pressure (hypertension) in humans by administering a compound of the formula I or a pharmaceutically usable acid additon salt of these compounds, possibly in addition to inert carrier materials and/or additives usual in pharmaceutical preparations in this field. The pharmaceutical preparations containing the compounds of the invention are for enteral, oral or rectal, as well as for parenteral administration, and, as stated, contain the pharmaceutically active ingredients alone or together with a conventional, pharmaceutically usable carrier material. The pharmaceutical presentation of the active ingredient is advantageously in the form of single doses, adapted to the desired method of administration, e.g. tablets, dragees, capsules, suppositories, granulates, solutions, emulsions, or suspensions. The dosage of the compounds is normally between 0.1 and 500 mg per dose, preferably between 1 and 150 mg per dose, and can be administered once or several times daily, preferably 2 to 3 times daily.

The preparation of the compounds of the invention is described in greater detail by the following examples. The given melting points were measured with a Buechi 510 melting point determination apparatus, are given in $^{O}C$ and are uncorrected. Mass spectra were determined with the Varian MAT-311-A (70eV) apparatus.

A. 6-/4-(1-Imidazolyl)-phenyl]-3-oxo-2.3.4.5-tetrahydropyridazine

(as an example of the preparation of a starting substance).

a) 4-/4-(1-Imidazolyl)-phenyl7-4-oxobutyric acid nitrile.

A solution of 10 g of 4-(1-imidazolyl)-benzaldehyde in 100 ml of dimethylformamide is added dropwise in a nitrogen atmosphere to a mixture of 0.23 g of sodium cyanide and 30 ml of dimethylformamide. There is then added a mixture of 2.4 g of acrylonitrile and 50 ml of dimethylformamide, and the mixture is stirred at $30^{O}C$ for 2 hours. After addition of water it is extracted with chloroform, the organic phase is washed with water, dried and evaporated, and the residue is recrystallized from ethanol.

Yield: 6.2 g; Mp. $205^{O}C$

MS /m/e7: 225 ($M^{+}$, 38%), 198 (3%), 171 (100%), 143 (43%), 116 (34%).

b) 4-/4-(1-Imidazolyl)-phenyl7-4-oxobutyric acid.

5.5 g of oxobutyric acid nitrile of a) is heated under reflux for 1 hour in 100 ml of 12 % hydrochloric acid. Active carbon and 100 ml of water are then added, the mixture brought to the boil and filtered hot. The filtrate is cooled and adjusted to pH 6, the precipitated acid is filtered off and dried.

Yield: 2.5 g; Decomposition Point $295^{O}C$.

MS /m/e7: 244 ($M^{+}$, 61%), 226 (31%), 198 (45%), 171 (100%), 143 (69%), 116 (68%).

c) 6-[4-(1-Imidazolyl)-phenyl]-3-oxo-2.3.4.5-tetrahydro-pyridazine.

1.4 g of oxobutyric acid of b) is stirred with 0.35 ml of hydrazine hydrate in 50 ml of water for 2 hours at 90°C. After cooling, the precipitated solid is filtered off, washed with water and dried.
Yield: 1.1 g; Mp. 219 to 220°C.
MS [m/e]: 240 ($M^+$, 100%), 211 (7%), 197 (6%), 183 (22%), 169 (16%), 142 (9%), 128 (6%), 115 (20%).

Example 1

4.5-Dihydro-6-[4-(1-imidazolyl)-phenyl]-3-mercapto-pyridazine.

A mixture of 2 g of 6-[4-(1-imidazolyl)-phenyl]-3-oxo-2.3.4.5-tetrahydropyridazine and 1.7 g of 2.4-bis-(4-methoxyphenyl)-2.4-dithioxo-1.3.2.4-dithiadiphosphetan (Lawesson reagent) in 100 ml of toluene are stirred at reflux temperature for 3 hours under an argon atmosphere. After cooling the mixture is filtered and the residue purified by column chromatography (silica gel/chloroform).
Yield: 0.3 g; Decomposition point 240°C.
MS [m/e]: 256 ($M^+$, 100%), 223 (18%), 196 (10%), 169 (16%).

Example 2

4.5-Dihydro-6-[4-(1-imidazolyl)-phenyl]-3-mercapto-5-methyl pyridazine.

A mixture of 6.9 g of 6-[4-(1-imidazolyl)-phenyl]-5-methyl-3-oxo-2.3.4.5-tetrahydropyridazine and 5.5 g of Lawesson reagent is stirred for 4 hours at 100°C in 100 ml of hexamethylphosphoric acid triamide under an argon atmosphere. After cooling, the reaction mixture is stirred into water, the precipitated solid filtered off, dried and recrystallized from toluene.

Yield: 4.8 g; Decomposition point 235°C.
MS /m/e/: 270 ($M^+$, 100%), 255 (15%), 237 (12%), 210 (4%), 170 (24%).

Example 3

4.5-Dihydro-6-/4-(1-imidazolyl)-phenyl/-3-mercapto-4-methyl pyridazine.

The reaction is carried out as in Example 1 with 2.3 g of 6-/4-(1-imidazolyl)-phenyl/-4-methyl-3-oxo-2.3.4.5-tetrahydropyridazine and 1.8 g of Lawesson reagent in 80 ml of toluene.
Yield: 0.7 g; Decomposition point 229 to 232°C.

Example 4

4.5-Dihydro-3-mercapto-5-methyl-6-/4-(2-methyl-1-imidazolyl)-phenyl/-pyridazine.

To a solution of 4 g of 6-/4-(2-methyl-1-imidazolyl)-phenyl/-5-methyl-3-oxo-2.3.4.5-tetrahydropyridazine in 80 ml of pyridine is added 3.3 g of diphosphorus pentasulphide at 70°C under an argon atmosphere, and the mixture stirred for 3 hours at 100°C. It is then evaporated to dryness, the residue shaken with 2N caustic soda solution and chloroform, and the aqueous phase neutralized with dilute hydrochloric acid. The precipitated solid is filtered off, washed with water and dried.
Yield: 0.7 g; Decomposition point 204 to 205°C.
MS /m/e/: 284 ($M^+$, 100%), 269 (19%), 251 (25%), 184 (38%).

Example 5

4.5-Dihydro-6-/4-(1-imidazolylmethyl)-phenyl/-3-mercapto-pyridazine.

The reaction is carried out as in Example 1 with 2 g of 6-/4-(1-imidazolylmethyl)-phenyl/-3-oxo-2.3.4.5-tetrahydro-pyridazine and 1.6 g of Lawesson reagent in 100 ml toluene.

Yield: 0.2 g; Decomposition point 198 to 203°C.
MS /m/e/: 270 ($M^+$, 49%), 203 (100%), 115 (76%).

Example 6

4.5-Dihydro-6-/5-(1-imidazolyl)-thien-2-yl/-3-mercapto-5-methylpyridazine.

The reaction is carried out as in Example 1 with 1 g of 6-/5-(1-Imidazolyl)-thien-2-yl/-5-methyl-3-oxo-2.3.4.5-tetrahydropyridazine and 0.78 g of Lawesson reagent in 50 ml of toluene.
Yield: 0.2 g; Decomposition point 233 to 235°C.
MS /m/e/: 276 ($M^+$, 100%), 261 (16%), 243 (14%), 203 (11%), 189 (20%), 176 (27%), 149 (20%).

Example 7

4.5-Dihydro-6-/4-(1-imidazolyl)-thien-2-yl/-3-mercapto-5-methylpyridazine.

The reaction is carried out as in Example 1 with 1 g of 6-/4-(1-imidazolyl)-thien-2-yl/-5-methyl-3-oxo-2.3.4.5-tetrahydropyridazine and 0.78 g of Lawesson reagent in 50 ml of toluene.
Yield: 0.2 g; Decomposition point 218 to 221°C.
MS /m/e/: 276 ($M^+$, 100%), 261 (31%), 189 (16%), 176 (27%), 149 (12%), 121 (16%).

Example 8

4.5-Dihydro-6-/5-(1-imidazolyl-methyl)-thien-2-yl/-3-mercaptopyridazine.

The reaction is carried out as in Example 1 with 2 g of 6-/5-(1-imidazolylmethyl)-thien-2-yl/-3-oxo-2.3.4.5-tetrahydropyridazine and 1.6 g of Lawesson reagent in 200 ml of toluene.
Yield: 0.4 g; Decomposition point 209 to 213°C.
MS /m/e/: 276 ($M^+$, 29%), 209 (74%), 147 (9%), 233 (29%), 68 (100%).

Example 9

6-/4-(1-Imidazolyl)-phenyl7-3-mercaptopyridazine.

A mixture of 4 g of 2.3-dihydro-6-/4-(1-imidazolyl)-phenyl7-3-oxopyridazine, 3.4 g of Lawesson reagent and 1oo ml of toluene is stirred at reflux temperature for 8 hours. It is then evaporated to dryness, the residue shaken with 2N caustic soda solution and chloroform and the aqueous phase neutralized with dilute hydrochloric acid. The precipitated solid is filtered off, washed with water and dried.

Yield: 1.2 g; Decomposition point 268 to 27o $^{o}$C.

MS /m/e7: 254 ($M^+$, 1oo%), 168 (49%), 141 (52%), 114 (34%).

Example 1o

6-/4-(1-Imidazolyl)-phenyl7-3-mercapto-5-methylpyridazine.

A mixture of 1.2 g of 2.3-dihydro-6-/4-(1-imidazolyl)-phenyl7-5-methyl-3-oxopyridazine, 1.1 g of diphosphorus pentasulphide and 5o ml of pyridine is stirred at reflux temperature for 2 hours. It is then evaporated to dryness, the residue shaken with 2N caustic soda solution and chloroform and the aqueous phase neutralized with dilute hydrochloric acid. The precipitated solid is filtered off, washed with water and dried.

Yield: o.2 g; Decomposition point 29o to 293 $^{o}$C.

MS /m/e7: 268 ($M^+$, 1oo%), 235 (11%), 182 (28%), 155 (32%), 128 (22%), 115 (19%).

Example 11

3-Mercapto-6-/4-(2-methyl-1-imidazolyl)-phenyl7-5-methylpyridazine.

A mixture of 5.4 g of 6-/4-(2-methyl-1-imidazolyl)-phenyl7-5-methyl-3-oxo-2.3.4.5-tetrahydropyridazine, 4.4 g of diphosphorus pentasulphide and 1oo ml of pyridine is stirred at 1oo $^{o}$C for 3 hours under atmospheric oxygen. It is then evaporated to dryness, the residue shaken with 2N caustic soda solution and chloroform, and the aqueous phase neutralized with dilute hydrochloric acid. The precipitated solid

is filtered off, dried and purified by column chromatography (silica gel/chloroform).
Yield: 1.5 g; Decomposition point 248°C.
MS /m/e7: 282 ($M^+$, 100%), 254 (5%), 249 (9%), 201 (6%), 169 (18%).

Example 12

6-/5-(1-Imidazolyl)-thien-2-yl7-3-mercapto-5-methylpyridazine.

The reaction is carried out as in Example 11 with 1 g of 6-/5-(1-imidazolyl)-thien-2-yl7-5-methyl-3-oxo-2.3.4.5-tetrahydropyridazine and 0.8 g of diphosphorus pentasulphide in 30 ml of pyridine.
Yield: 0.12 g; Decomposition point 265 to 267°C.

Example 13

6-/4-(1-Imidazolyl)-phenyl7-3-methylthiopyridazine.

To a suspension of 0.15 g of sodium hydride (80% in paraffin oil) in 60 ml of dimethylformamide a solution of 1.5 g of 4.5-dihydro-6-/4-(1-imidazolyl)-phenyl7-3-mercaptopyridazine in 100 ml of dimethylforamide is added dropwise over 15 minutes. After stirring for 30 minutes at 110°C, 0.86 g of methyl iodide is added and the mixture stirred at reflux temperature for 2 hours. It is then evaporated, and the residue shaken with water and chloroform. The chloroform phase is extracted with 2N hydrochloric acid, the aqueous phase neutralized with dilute caustic soda solution and extracted with chloroform. The chloroform phase is dried, evaporated and the residue purified by column chromatography (silica gel/chloroform).
Yield: 0.4 g; Decomposition point 194°C.
MS /m/e7: 268 ($M^+$, 100%), 181 (10%), 168 (12%), 141 (13%).

Example 14

6-/4-(1-Imidazolyl)-phenyl7-5-methyl-3-methylthiopyridazine.

The reaction is carried out as in Example 13 with 3 g of 4.5-dihydro-6-/4-(1-imidazolyl)-phenyl7-3-mercapto-5-methyl-pyridazine, o.35 g of sodium hydride and o.7 g of methyl iodide in 15o ml of dimethylformamide.

Yield: o.4 g; Decomposition point 154°C.

MS /m/e7: 282 ($M^+$, 1oo%), 237 (6%), 195 (1o%), 182 (14%), 17o (1o%), 155 (1o%).

Example 15

5-Methyl-6-/4-(2-methyl-1-imidazolyl)-phenyl7-3-methylthio-pyridazine.

The reaction is carried out as in Example 13 with o.5 g of 3-mercapto-5-methyl-6-/4-(2-methyl-1-imidazolyl)-phenyl7-pyridazine, o.06 g of sodium hydride and o.25 g of methyl iodide in 2o ml of dimethylformamide.

Yield: o.1 g; Decomposition point 164 to 166°C.

MS /m/e7: 296 ($M^+$, 1oo%), 262 (6%), 251 (6%), 2o9 (8%), 169 (12%), 128 (11%), 115 (12%).

Example 16

4.5-Dihydro-6-/4-(1-imidazolyl)-phenyl7-3-mercapto-5-methyl-pyridazine hydrochloride.

1 g of 4.5-dihydro-6-/4-(1-imidazolyl)-phenyl7-3-mercapto-5-methylpyridazine is suspended in ethanol. Ethanolic hydrochloric acid is then added and the salt is precipitated by addition of ether to the ethanolic solution. The product is dried after filtration.

Yield: o.3 g; Decomposition point 284 to 286°C.

Fumarates, acetates, benzoates etc., are prepared similarly to Example 16.

PATENT CLAIMS:

1. Substituted 3-mercaptopyridazines und their 3-alkylthio derivatives of the general formula I

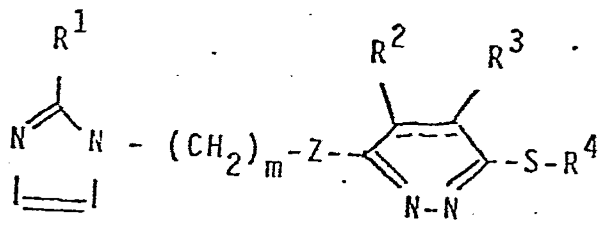

I

in which $R^1$, $R^2$, $R^3$ and $R^4$ each and independently are members selected from the group consisting of hydrogen and the $C_{1-4}$-lower alkyl residues,

m represents cero or a whole number from 1 to 5.

Z represents a member selected from the group consisting of phenylene and thienylene, and

---- signifies a double or single bond between 2 carbon atoms,

and the pharmacologically acceptable acid addition salts thereof.

2. Compounds as claimed in claim 1, wherein
$R^4$ is hydrogen,
m is cero or 1,
Z is 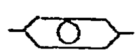, and
---- signifies a single bond,
said compounds existing in equilbrium with the corresponding tautomeric 4.5-dihydro-3(2H)-pyridazine thiones, and the pharmacologically acceptable acid addition salt thereof.

3. Compounds as claimed in claim 1, wherein
$R^4$ is hydrogen,
m is cero or 1,
Z is a member selected from the group consisting of

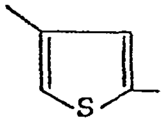 and 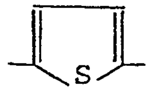 , and

---- signifies a single bond,

said compounds existing in equilibrium with the corresponding tautomeric 4.5-dihydro-3(2H)-pyridazine thiones, and the pharmacologically acceptable acid addition salts thereof.

4. Compounds as claimed in claim 1, wherein

$R^4$ represents hydrogen,

m is cero or 1,

Z signifies 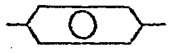, and

---- signifies a double bond,

said compounds existing in equilibrium with the corresponding tautameric 3(2H)-pyridazine thiones, and the pharmacologically acceptable acid addition salts thereof.

5. Compounds as claimed in claim 1, wherein

$R^4$ represents hydrogen,

m is cero or 1,

Z represents a member selected from the group consisting of

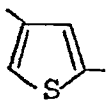 and 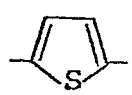, and

---- signifies a double bond,

said compounds existing in equlibrium with the corresponding tautomeric 3(2H)-pyridazine thiones, and the pharmacologically acceptable acid addition salts thereof.

6. Compounds as claimed in claim 1, wherein

$R^4$ is a member selected from the group consisting of the $C_{1-4}$-lower alkyl residues,

m is cero or 1,

Z is a member selected from the group consisting of

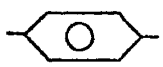 , 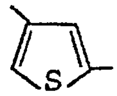 and 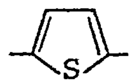 , and

==== signifies a double bond, and

the pharmacologically acceptable acid addition salts thereof.

7. Process for the preparation of compounds of the formula 1 with $R^4$ = hydrogen, according to claims 1-3, characterized in that 3-oxo-2,3,4,5-tetrahydropyridazines of the formula II

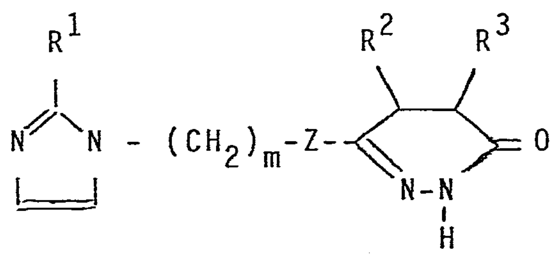

II

in which $R^1$, $R^2$, $R^3$, m, Z have the meanings given in formula I, is reacted with a suitable sulphiding agent, e.g. diphosphorus pentasulphide or 2,4-bis-(4-methoxyphenyl)-2,4-dithioxo-1,3,2,4-dithiadiphosphetan (Lawesson reagent), under a protective gas atmosphere in a suitable organic solvent, e.g. benzene, toluene, chloroform, pyridine, hexamethylphosphoric acid triamide.

8. Process for the preparation of compounds of the formula I with $R^4$ = hydrogen, according to claims 1, 4, 5, characterized in that a compound of the formula II is reacted with a suitable suphiding agent, e.g. diphosphorus pentasulphide or Lawesson reagent, in the presence of an oxidizing agent, e.g. atmospheric oxygen, in a suitable organic solvent, e.g. benzene, toluene, chloroform, pyridine, hexamethylphosphoric acid triamide.

9. Process for the preparation of compounds of the formula I with $R^4$ = hydrogen, according to claims 1, 4, 5, characterized in that a 2,3-dihydro-3-oxopyridazine of the formula VII .

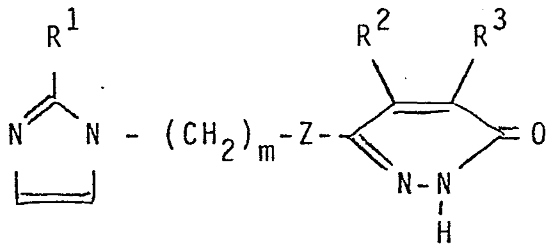

II

in which $R^1$, $R^2$, $R^3$, m, Z have the meanings given in formula I, is reacted with a suitable sulphiding agent e.g. diphosphorus pentasulphide or Lawesson reagent, in a suitable organic solvent, e.g. benzene, toluene, chloroform, pyridine, hexamethylphosphoric acid triamide.

10. Process for the preparation of compounds of the formula I with $R^4$ = alkyl, according to claims 1, 6, characterized in that a 4,5-dihydro-3-mercaptopyridazine of the formula I with $R^4$ = hydrogen according to claims 1-3, is reacted with an alkylating agent of the formula VIII

$$X-R^4 \qquad VIII$$

in which $R^4$ is a $C_{1-4}$-lower alkyl residue and X is a suitable leaving group, e.g. chloride, bromide, iodide, methylsulphate, tosylate, in the presence of an oxidizing agent, e.g. atmospheric oxygen, or a 3-mercaptopyridazine of the formula I with $R^4$ = hydrogen according to claims 1, 4, 5, is reacted with said alkylating agent in a suitable organic solvent, e.g. dimethylformamide, dimethylsulphoxide, hexamethylphosphoric acid triamide, with optional use of an auxiliary base, e.g. sodium hydride or potassium carbonate.